# EUROPEAN PATENT APPLICATION

(11) **EP 0 899 260 A1**
(43) Date of publication of application: **03.03.1999**
(21) Application number: 98202889.6
(22) Date of filing: 20.09.1992
(51) Int. Cl.: C07D 211/46, C08K 5/378

(54) **Stabilizers for organic materials**

(30) Priority: 25.09.1991 NL 9101619
(62) Divisional of application: 92202942.6
(71) Applicant: AKCROS CHEMICALS V.O.F., NL-6041 KV Roermond (NL)
(72) Inventor: Schmets, Gerard Hubert Frans, 6085 AV Horn (NL); Kroon, Erica Gertruda Arnolda, 6071 XM Swalmen (NL); Peters, Frans Jeanette Maria, 6061 EL Posterholt (NL); Overveem, Jan Cornelis, 3925 KN Scherpenzeel (NL)
(74) Representative: De Hoop, Eric

(57) **Abstract**

The invention relates to a stabilizer for organic materials which are liable to oxidative and/or thermal decomposition and/or decomposition under influence of actinic irradiation, of formula 1, wherein R₄ and R₅ which may be the same or different, represent an optionally branched alkylene group having 1-8 carbon atoms.

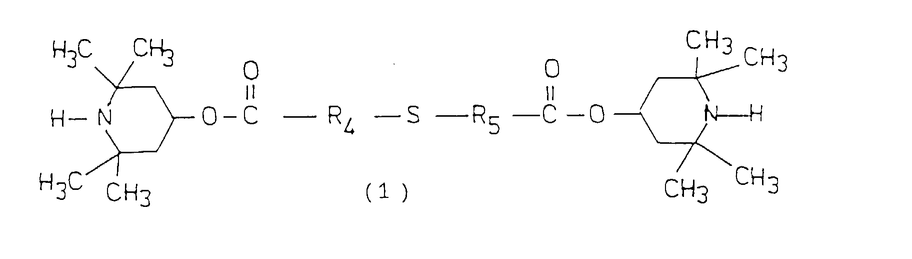

## Description

The invention relates to novel stabilizers for organic materials which are liable to oxidative and/or thermal decomposition and/or decomposition under influence of actinic irradation, such as petroleum products, e.g. lubricating oils and lubricating greases, and plastics, such as polyvinylchloride, polyalkylenes and polystyrene.

In order to prevent oxidative and/or thermal decomposition (degradation) of petroleum products and plastics, such as polyvinylchloride, polyalkylenes and polystyrene, on large scale antioxidants and antiozonants are used, which depending on their chemical structure, inhibit the initiation or propagation of oxidation reactions. Especially as antioxidants that inhibit the propagation of oxidation reactions, secondary alkylaryl- and diarylamines and, in particular, hindered phenols are applied (vide Kirk-Othmer, Encyclopedia of Chemical Technology, 3rd edition part 3, page 128-148 and in particular pages 130-133).

In order to prevent decomposition under influence of actinic irradiation, in particular decomposition under influence of UV-irradiation, often so-called HALS (hindered amine light stabilizers) are applied, the most important ones of which are 2,2,6,6-tetramethyl-4-piperidinol and carboxylic acids, such as bis-(2,2,6,6-tetramethyl-4-piperidinyl)sebacate and -azelate, which are especially active in polypropylene, polyethylene, styrene polymers, polyurethanes and coatings containing acrylic polymer (vide Kirk-Othmer, Encyclopedia of Chemical Technology, 3rd edition, volume 23, pages 615-627 and in particular pages 620-621 and page 624).

Although said compounds generally have good properties, continuously novel compounds of these types are developed, in order to meet the higher and higher requirements with respect to the stability of petroleum products (e.g. lubricating oil) and of polymers.

In EP-A-88108282.0 (publ. no. 300160) the ester of 2,2,6,6-tetramethyl-4-piperidinol and behenic acid is described, which serves as stabilizer against the decomposition of polymers by actinic irradiation.

Now the object of the invention is to provide novel stabilizers with particularly favourable, improved properties.

Accordingly novel compounds of formula 1 are provided 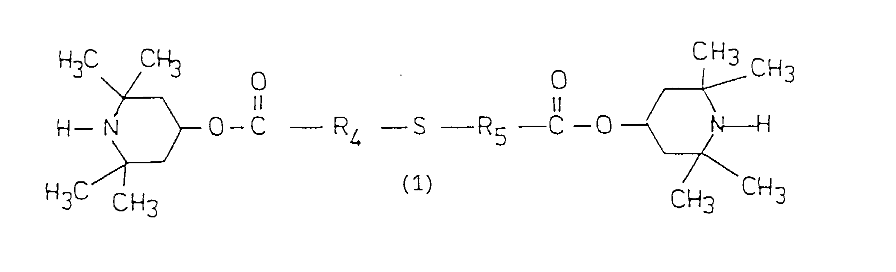 wherein R₄ and R₅, which may be the same or different, may represent an optionally branched alkylene group having 1-8 C-atoms.

These compounds have a stabilizing effect both against thermal and oxidative degradation and against decomposition or degradation under influence of actinic irradiation, in particular UV-irradiation. So these are stabilizers with combined antioxidant/light stabilizer properties.

Preferably at least one of the groups R₄ and R₅ is an ethylene or 1-methylethylene (propylene) group. In that case the compounds are bis esters of thiodipropionic acid or thiodiisobutyric acid, which may be easily prepared, have a good stability theirselves and provide an optimum stabilizing effect.

The novel stabilizers are included in usual amounts in the petroleum products or plastics to be stabilized that is to say amounts of 0.1-10 and preferably 0.05-5% by weight. Optimal results are generally obtained with 0.1-3 and in particular with 1-3% by weight.

The invention is further elucidated by the following examples.

### Example I

### Preparation of thiodipropionic acid bis (2,2,6,6-tetramethyl-4-piperidinyl ester) (formula 2)

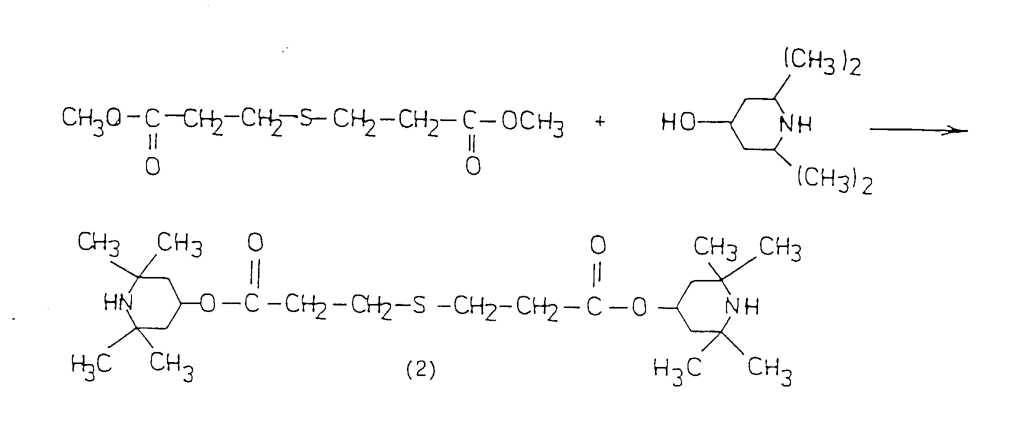

A three neck-flask with stirrer, thermometer and cooler is filled with:
10.3 g (0.05 mole) thiodipropionic acid dimethyl ester
15.7 g (0.1 mole) 2,2,6,6-tetramethyl-4-piperidinol
150 ml toluene
1 g titanium butoxide

The reaction mixture was heated for 8 hours at reflux temperature.
During this period about 500 ml of toluene was distilled and at the same time replaced by fresh toluene. After the reflux period the reaction mixture was cooled down and washed with water. The remaining toluene solution was dried and filtered. After distilling the toluene a substantially colourless viscous liquid was obtained.
Yield: 21 g = 92%

From the NMR-spectrum it follows, that this viscous liquid substantially consists of the compound of formula 2.

### Example II

The compound according to Example I was applied as stabilizer in a plasticizer-free, calcium-zinc-stabilized PVC composition

| | |
|---|---|
| S-PVC (K=68-70) | 100 parts by weight |
| Paraloid KM-334 (Impact-strength additive | 5-8 parts by weight |
| Paraloid K-120N (Flow improver) | 0.5-1.5 parts by weight |
| Hydrocarb 95T (chalk) | 3-10 parts by weight |
| Titanium dioxide (Kronos-2220) | 3-10 parts by weight |
| Ca/Zn stabilizer | 3.5-4.5 parts by weight |
| Stabilizer (oxyd/therm., UV) | 1-3 parts by weight |

Test plates of this composition which contain the stabilizer of Example I, and test plates which contain a comparative stabilizer β-(3,5-di-t.butyl-4-hydroxyphenyl)propionic acid-n-octadecyl ester, were tested on their light stability with the aid of an apparatus for artificial exposure, QUV panel, lamp A.

The light stability with the stabilizer of Example I was, when applied in amounts between 1 and 3 (on 100 parts by weight of PVC), in any case better than with the comparative stabilizer.

### Example III

The compound according to Example I was applied as stabilizer in a plasticizer containing film composition stabilized with Ba/Zn:

| | |
|---|---|
| S-PVC (K=65) | 100 parts by weight |
| Plasticizer (DOP) | 20-25 parts by weight |
| Epoxized soy bean oil | 2-3 parts by weight |
| Titanium dioxide | 5-10 parts by weight |
| Stabilizer | 1.5-3 parts by weight |

Test films of this composition which contain the stabilizer of Example I, upon measuring the light stability with the artificial exposing apparatus QUV panel, lamp A, provide a better result than similar films which contain a corresponding amount of a usual stabilizer penta-erythritol-tetra[β-3-(3,5-di-t-butyl-4-hydroxyphenyl)propionate].

## Claims

1. Stabilizer for organic materials which are liable to oxidative and/or thermal decomposition and/or decomposition under influence of actinic irradiation, of formula 1, 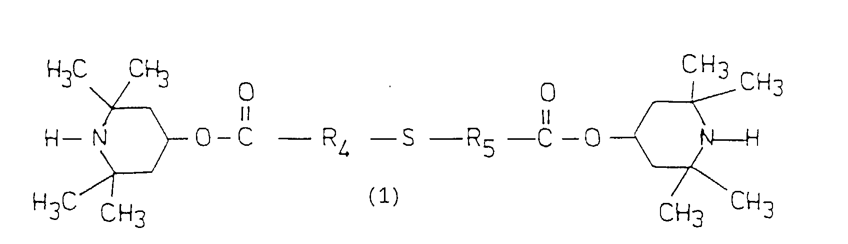 wherein R₄ and R₅ which may be the same or different, represent an optionally branched alkylene group having 1-8 carbon atoms.

2. Stabilizer according to claim 1, wherein at least one of the groups R₄ and R₅ is an ethylene or 1-methylethylene group (propylene group).

3. Stabilizer according to claim 2, wherein R₄ and R₅ both are an ethylene group.

4. Stabilizer according to claim 2, wherein R₄ and R₅ both are a 1-methylethylene group.

5. Petroleum product or plastic material stabilized against oxidative and/or thermal decomposition and/or decomposition under influence of actinic irradiation, characterized in that it contains a compound according to any one of the preceding claims as stabilizer.
